# EUROPEAN PATENT SPECIFICATION

(11) **EP 4 217 033 B1**
(45) Date of publication and mention of the grant of the patent: **16.04.2025**
(21) Application number: 21795098.9
(22) Date of filing: 24.09.2021
(51) Int. Cl.: A61M 16/00, A61M 16/06, A61M 16/08

(54) **BREATH SENSING WITH REMOTE PRESSURE SENSOR**
ATEMERFASSUNG MIT FERNDRUCKSENSOR
DÉTECTION DE RESPIRATION AVEC CAPTEUR DE PRESSION DISTANT

(30) Priority: 25.09.2020 US 202063083388 P
(43) Date of publication of application: 02.08.2023
(73) Proprietor: Vapotherm, Inc., Exeter, NH 03833 (US)
(72) Inventor: LEONARD, Scott A., Exeter, NH 03833 (US)
(74) Representative: Larsen, Charles
(86) International application number: PCT/US2021/051987
(87) International publication number: WO 2022/067061

(56) References cited:
- EP-A1- 3 646 913
- WO-A1-2009/094532
- WO-A1-2019/191814
- WO-A1-98/18513
- WO-A2-2006/138579
- US-A- 5 099 836
- US-A1- 2007 175 473
- US-A1- 2009 101 147
- US-A1- 2009 253 995
- US-A1- 2013 092 165

## Description

### Background

Patients with respiratory ailments are often treated with respiratory assist devices that deliver supplemental breathing gas to the patient. Such devices may deliver gas to the patient using high flow therapy (HFT). HFT devices deliver breathing gas at a high flow rate via an interface, such as a nasal cannula, to increase the patient's fraction of inspired oxygen (FiO₂), decrease the patient's work of breathing, or accomplish both. Increasing FiO₂ or decreasing the work of breathing helps the patient recover from respiratory ailments, such as respiratory distress or bronchospasms. Some HFT devices heat and humidify the delivered breathing gas for medical reasons (e.g., to maintain the pliability of the tissues of surfactant-deficient patients, or to preserve mucosal integrity) or to reduce patient discomfort.

In order to make respiratory clinical decisions for a patient, a clinician needs as much information as possible about the patient's current and historical medical status. For example, there are several methods available for measuring breathing, but many do so indirectly by looking at changes to the patient's heart rate, blood, or muscle movement. Conventionally, breath sensing measurements are available for patients receiving respiratory therapies that use a sealed mask, such a CPAP, BiPAP, mechanical ventilator, or other therapies. With a mask, accurate sensing is straightforward, because the mask seal delimits a closed system. But with high flow and high velocity nasal cannula systems, these measurements become more challenging, as the patient's nares are open to the environment. The flow rate from the cannula may exceed the patient's breathing flow rate and obscure the signal of pressure or temperature. Due to the open nature of the cannula interface, the pressure changes are very small, for example, on the order of 5 to 50 Pa, which are difficult to detect. Additionally, issues related to patient comfort are more problematic in the open nasal cannula systems and in systems which provide humidified breathing gas.

In U.S. Publication No. 2013/0092165, a nasal cannula ventilation system is described for treating lung disease or for exercise conditioning, incorporating a Venturi system. The ventilation cannula comprises unique positioning features to positively locate a gas delivery nozzle in an optimal location to optimize Venturi performance, patient comfort and fitment to the patient. U.S. Patent No. 5,099,836 describes a system for delivering oxygen on demand to a user, comprising a pressure transducer for sensing pressures during the breathing cycle of a user and for creating an electrical signal proportional to said sensed pressures, an electronic amplifier for amplifying the signal to at least 10,000 times the original signal strength, electronic filters for regulating the amplitude of the signal and for correcting the point of the signal where expiration stops and inspiration begins to correspond to zero respiratory pressure, an on/off switching circuit and cooperating valve drive circuit for switching to an on condition in response to a preselected first pressure and to an off condition in response to a preselected second pressure, and a valve driven by the valve drive circuit means for directing oxygen from an oxygen source to a user. WO 2009/094532 describes a nasal cannula, for supplying a respiratory gas to a patient, a respiratory therapy system incorporating the nasal cannula, a method of treating a patient with sleep disorder by using the nasal cannula, a diagnostic tool for measuring nasal cavity pressure of a patient, and a method of using the diagnostic tool for measuring nasal cavity pressure of a patient. U.S. Publication No. 2009/0253995 describes a method, system and apparatus for determining respiratory volume flow rate of a subject and associated parameters such as tidal volume, minute volume, and respiratory rate. The method for determining respiratory volume flow rate of a subject can include selecting an airway cavity of the subject, measuring delivery volume flow rate of respiratory gas delivered to the airway cavity, measuring pressure within the airway cavity and calculating a respiratory volume flow rate of the subject using the measured delivery volume flow rate of respiratory gas delivered to the airway cavity and the measured pressure within the airway cavity. U.S. Publication No. 2007/0175473 describes a high flow therapy system including a microprocessor, a heating element a non-sealing respiratory interface and a sensor. The sensor is disposed in electrical communication with the microprocessor and is configured to measure pressure in an upper airway of the patient. U.S. Publication No. 2009/0101147 describes a device, system and method for providing high flow therapy interfaces for use in the treatment of respiratory conditions and in assisted respirations. A nasal cannula for delivery of respiratory gases can include at least one nasal insert and at least one flange coupled to the at least one nasal insert where the at least one flange is configured to partially impede the egress of respiratory gasses delivered to an upper airway of a patient is provided. An oral interface for delivery of high flow therapy to the upper airway of a patient can include a gas diffuser that includes a terminus housing having a plurality of holes to allow the delivery of respiratory gases. WO 98/18513 describes an apparatus that conserves oxygen delivered from a supply to a patient through a cannula by providing oxygen delivery selectively in accordance with the physiological requirements and current breathing pattern of the patient. An oxygen conserving unit includes a controller that operates responsive to timed relationships among pressure signals determined by a fuzzy logic program to deliver oxygen to the patient by opening a valve when a sensed pressure in the patient's nasal passage reaches a threshold level and when the controller determines that the reaching of the threshold is indicative of an inhalation cycle. WO 2006/138579 describes a method of forming a cannula from a cannula mandrel assembly comprising a pair of mouthpiece/nasal mandrels and a mating facepiece mandrel.

### Summary

Devices and methods are provided herein for sensing and monitoring of patient breathing during high flow respiratory therapy. According to one aspect, there is provided a breath sensing device according to claim 1. This aspect may also provide an apparatus according to claim 11. The device includes a nasal cannula having a sensing lumen and pressure sensor for breath sensing that can provide an accurate and clear signal while high flow rates of breathing gas are delivered from the nasal cannula, for example, at a high exit velocity. A length of tubing may be used to consistently convey the pressure changes to the pressure sensor from near the nares. The pressure sensor may be located remotely, and the tubing may be used to convey the pressure change between the nares and the remotely positioned sensor. From the pressure measurements, certain characteristics of the patient, such as breathing pattern or efficiency of breathing, can be determined and used to track the clinical status of the patient or to adjust the therapy.

Directly measuring air flow of patient breathing is preferable relative to conventional methods that indirectly assess or detect patient breathing via other physiological indicators. By locating the pressure sensor away from the nares of the patient, for example, at the end of a small-bore sensing lumen, pressure changes in the lumen that result from patient inspiration and expiration are reduced to lower frequencies that are more easily detected by the sensor, avoiding significant signal noise due to gas turbulence. Furthermore, condensation can be removed from the sensing lumen by applying a constant, pulsatile, or burst flow of purge gas. This can be beneficial in systems where the tubing is prone to occlusion by condensation, especially when the therapy is humidified. Capillary action tends to draw water into the tubing where it can mask the small pressure changes and damage the sensor.

The breath sensing apparatus can be integrated in a respiratory system with a breathing gas source or can be packaged as a separate device capable of adapting to a variety of systems. Information about a patient's breathing can be useful in several ways. A high flow or high velocity nasal cannula therapy device could make use of this data to report the information, adjust therapy, or ensure proper use of the device.

The nasal cannula for breath sensing of a patient being provided high flow respiratory therapy includes a cannula body having a first connector configured to receive a first flow of breathing gas and a second connector configured to receive a second flow of breathing gas; a first nasal prong configured to deliver the first flow of breathing gas to a nare of the patient; a second nasal prong configured to deliver the second flow of breathing gas to a nare of the patient; and a pressure sensor system positioned on the cannula, on or near one or more of the nasal prong exits such that, during patient inspiration and expiration, the pressure of gas flowing in and out of the nare can be detected. The nasal cannula is configured with a first sensing lumen with a first sensing tip positioned along the first nasal prong, such that gas flows entering and exiting the nares flow over the first sensing tip to create a pressure change within the first sensing lumen. A first pressure sensor is also provided in the nasal cannula, the sensor being configured to detect a first pressure in the first sensing lumen. The pressure sensor is positioned at a proximal end of the first sensing lumen opposite the first sensing tip. A cannula configured with such a pressure sensor system can provide the advantage of deriving information about a patient's breathing while functioning with high velocity or high flow nasal cannula therapy. The remote positioning of the pressure sensor and use of a sensing lumen with distal tip allows for detection of pressure changes without substantial turbulence, noise, or high frequency pressure fluctuations at the pressure sensor, thus overcoming obstacles resulting from the open nature of nasal cannula therapy.

In some implementations, multiple pressure sensor systems may be used. For example, the nasal cannula may further include a second sensing lumen having a second sensing tip positioned along the second nasal prong such that, during patient inspiration and expiration, gas flows over the second sensing tip to create a second pressure change within the second sensing lumen; and a second pressure sensor configured to detect a second pressure in the second sensing lumen and positioned at a proximal end of the second sensing lumen opposite the second sensing tip. In some implementations, the first sensing lumen includes a housing mounted on the cannula body, and a tube configured to enable fluid communication between the first sensing tip and the pressure sensor, wherein the tube is in fluid communication with the pressure sensor at the proximal end of the first sensing lumen, and wherein the housing is fluidically connected to the tubing. The tube may have a length between about 5 mm and about 3 m and an internal diameter between about 0.25 mm and about 2.5 mm.

In some implementations, a control gas flows from a flow generator through at least a portion of the pressure sensing tube in a direction from the proximal end to the sensing tip. The control gas collides with patient breath at a boundary region adjacent to the first sensing tip. The control gas may be delivered continuously or intermittently as a bolus flow. In some implementations, the control gas has a flow rate between about 0.1 mL/min and about 10 mL/min. The flow generator may be one of a fan, a syringe pump, a blower, a compressor, a bellows, or a wall air source. In some implementations, the nasal cannula is configured with the control gas so as to purge condensation from one or more sensing lumens. In some implementations, a sensing lumen includes a tee joint positioned along the tube and configured to convey the control gas into the tube from the flow generator. In some implementations, control gas is provided for first and second sensing lumens. In some implementations, the nasal cannula further includes a temperature sensor configured to measure a breath temperature.

In some implementations, the detected pressure by the pressure sensing system indicates a difference between an internal pressure within the first sensing lumen and ambient pressure of the surrounding environment. The difference may be about 1 Pa to about 5 Pa. The difference may be at least about 1 Pa or at least about 5 Pa. In some implementations, the first pressure sensor is configured to output a pressure signal indicative of the detected first sensing lumen pressure. A second pressure sensor may be similarly configured.

In some implementations, the first sensing tip has an exit port that is angled relative to a longitudinal axis of the first sensing lumen. The exit port may include a tip face at an angle other than 90 degrees relative to the longitudinal axis of the sensing lumen. For example, the angle may be between about 30 degrees and about 60 degrees. In some implementations, the angle is about 45 degrees. In some implementations, the angle is about 30 degrees or about 60 degrees. In some implementations, the tip face has rounded, beveled, or chamfered edges. In some implementations, the sensing tip is positioned on an exterior surface of the first nasal prong. In some implementations, the sensing tip is positioned on a dorsal side of the exterior surface of the first nasal prong. In some implementations, the sensing tip is positioned proximally to a prong tip of the first nasal prong. In some implementations, the sensing tip is positioned on the nasal cannula such that the sensing tip resides within the nare of the patient while the cannula is in use. The sensing tip may have an internal diameter between about 0.25 mm and 2.5 mm. In some implementations, the first nasal prong has an internal diameter of about 1.0 mm to about 4.0 mm. For example, the ratio of sensing tip diameter to nasal prong diameter is between about 0.0625 and about 2.5.

The breath sensing device includes a controller configured to receive a pressure signal from the first pressure sensor indicative of the first sensing lumen pressure and convert the pressure signal into data. In addition to the advantages of the nasal cannula recited above, the breath sensing device is advantageous as an adaptable device that can provide breath sensing and monitoring capabilities to existing respiratory therapy systems.

In some implementations, the device is configured to couple to a respiratory therapy system. The controller may be configured to send instructions to the respiratory therapy system to change an operating parameter of the respiratory therapy system, or the controller may be configured to send the data to the respiratory therapy system for determining a change to an operating parameter of the respiratory therapy system. The respiratory therapy system is configured to provide the first flow of breathing gas to the nasal cannula.

In some implementations, the controller is configured to determine periods of patient inspiration and/or expiration based on the data from the first pressure sensor. The controller may be configured to determine and display at least one of patient expiratory/inspiratory ratio, a current breath rate, or a patient breathing pattern based on the data from the first pressure sensor.

The controller is configured to apply an algorithm to the data. The algorithm is configured to determine an average pressure in the data, determine maximum and minimum peaks in the data and/or filter the data, determine an average maximum peak over a period of time, determine an average minimum peak over a period of time, and determine a pressure threshold as a percentage of the average maximum peak. The algorithm is configured to identify a breath when the pressure crosses from below the average pressure to above the average pressure, then exceeds the average pressure by at least the pressure threshold, then reaches a maximum peak, then crosses from above the average pressure to below the average pressure, then reaches a minimum peak, and then crosses from below the average pressure to above the average pressure. In some implementations, the algorithm is configured to identify and exclude non-breathing periods from the data.

In some implementations, the nasal cannula further includes a second sensing lumen having a second sensing tip positioned along the second nasal prong such that, during patient inspiration and expiration, gas flows over the second sensing tip to create a second pressure change within the second sensing lumen. In some implementations, the nasal cannula further includes a second pressure sensor configured to detect a second pressure in the second sensing lumen and positioned at a proximal end of the second sensing lumen opposite the second sensing tip. The controller may be configured to average data from the first pressure sensor and the second pressure sensor to generate an average sensing lumen pressure. The controller may be configured to receive data from only one of the first pressure sensor or the second pressure sensor when one of the nares of the patient is blocked.

Where provided, the respiratory therapy system may include a capital unit including a pressurized gas source, and a delivery tube configured to convey the first flow of breathing gas from the capital unit to the first connector of the nasal cannula. The respiratory therapy system may further include a controller configured to receive a pressure signal from the first pressure sensor indicative of the first sensing lumen pressure and convert the pressure signal to data. In addition to the advantages of the nasal cannula recited above, the respiratory therapy system is advantageous by allowing for feedback control of the respiratory therapy provided by the capital unit based on the information derived from the breath sensing data.

In some implementations, the controller is configured to change an operating parameter of the capital unit based on the received data. In some implementations, the controller is configured to determine periods of patient inspiration and/or expiration based on the data from the first pressure sensor. In some implementations, the controller is configured to determine and display a patient expiratory/inspiratory ratio, current breath rate, and/or a patient breathing pattern based on the data from the first pressure sensor.

In any of the above aspects, the first flow of breathing gas may be heated and humidified (e.g., by a vapor transfer unit or a hotpot humidifier). The first flow of breathing gas may be configured to exit the first nasal prong at a high velocity, for example, at an exit velocity of at least about 40 m/s and/or to be delivered at a flow rate between about 1 L/min and 60 L/min.

In another aspect, there is provided a method according to claim 14, for sensing patient breathing when the patient is provided with high flow respiratory therapy. The method includes the step of detecting a pressure in a sensing lumen of the nasal cannula using a pressure sensor positioned at a proximal end of the sensing lumen opposite a tip of the sensing lumen. The sensing lumen is positioned such that, during patient inspiration and expiration, gas flows over the tip of the sensing lumen to create a pressure change within the sensing lumen. The remote positioning of the pressure sensor and use of a sensing lumen with distal tip allows for detection of pressure changes without substantial turbulence, noise, or high frequency pressure fluctuations at the pressure sensor, thus overcoming obstacles resulting from the open nature of nasal cannula therapy.

The method further includes transmitting a signal indicative of the detected pressure from the pressure sensor to a controller, and processing the signal using an algorithm stored in a memory of the controller. Processing the signal includes determining a current breath rate, and the method further includes displaying the current breath rate. The signal is indicative of a plurality of pressures detected by the pressure sensor over a period of time, and processing the signal includes identifying peaks in the plurality of detected pressures over time. The method may further include determining an increase or decrease in pressure over time. The method may further include identifying an apnea event based on the signal and logging the apnea event.

The method further includes determining an average pressure in the data. The method further includes determining maximum and minimum peaks in the data and/or filtering the data. The method further includes determining an average maximum peak over a period of time, determining an average minimum peak over a period of time, and determining a pressure threshold as a percentage of the average maximum peak. The method further includes determining a breath when the pressure crosses from below the average pressure to above the average pressure, then exceeds the average pressure by at least the pressure threshold reaches a maximum peak, then crosses from above the average pressure to below the average pressure, then reaches a minimum peak, and then crosses from below the average pressure to above the average pressure.

the method may further include operating the controller to alter an operating parameter of the respiratory therapy based on the detected pressure. The operating parameter may be at least one of a breathing gas flow rate, an oxygen percentage of the flow of breathing gas, a breathing gas temperature, a breathing gas humidity, or an aerosolized medicament flow rate. In some implementations, the detected pressure is a differential pressure between a pressure within the sensing lumen and an ambient pressure. In some implementations, the method further includes conveying a control flow of gas through the sensing lumen from a flow generator. The method may further include purging condensation from the sensing lumen with the control flow of gas. In some implementations, the method further includes humidifying the flow of breathing gas using a vapor transfer unit or a hotpot humidifier.

In another aspect, outside the scope of the claims, there is provided a method of manufacturing a nasal cannula for respiratory therapy and breath sensing. The method includes the step of maintaining a first mandrel, a second mandrel, and a third mandrel in a fixed arrangement with respect to each other, wherein the third mandrel is positioned along at least a portion of the first mandrel at a distance from the first mandrel; coating the arrangement with a material, The method includes the step of curing the coated arrangement; trimming at least one coated mandrel to create an opening in the coating of the trimmed mandrel. The method includes the step of removing the cured coating from the arrangement, wherein the coating on the first, second, and third mandrels form a first nasal prong, a second nasal prong, and a sensing lumen, respectively.

The step of coating may include immersing the arrangement in the material and removing the arrangement from the material. In some implementations, the step of coating includes spraying the material onto the arrangement. In some implementations, the first, second, and third mandrels are fixedly held on a substrate.

### Brief Description of the Drawings

The foregoing and other objects and advantages will be apparent upon consideration of the following detailed description, taken in conjunction with the accompanying drawings, in which like reference characters refer to like parts throughout, and in which:
FIG. 1 shows a nasal cannula with a breath sensing lumen, according to an illustrative implementation;
FIG. 2 shows a flowchart outlining a method for breath sensing during respiratory therapy, according to an illustrative implementation;
FIG. 3 shows a flowchart outlining a method of manufacturing of a breath sensing nasal cannula, according to an illustrative implementation; and
FIG. 4 shows a block diagram of a respiratory therapy system configured to monitor patient breathing, according to an illustrative implementation.

### Detailed Description

To provide an overall understanding of the assemblies and methods described herein, certain illustrative implementations will be described. These illustrative implementations are described for high velocity respiratory therapy, which can be understood to mean that breathing gas is delivered to the patient at a velocity of at least about 40 m/s, for example, exiting from a nasal cannula prong. Although the implementations and features described herein are specifically described for high velocity respiratory therapy, it will be understood that all the components and other features outlined below may be combined with one another in any suitable manner and may be adapted and applied to other respiratory therapy systems and devices, including low flow oxygen therapy, continuous positive airway pressure therapy (CPAP), mechanical ventilation, oxygen masks, Venturi masks, or Tracheostomy masks, or combinations thereof. Although the features are described within regard to over-ear lariat-type nasal cannula securement systems, it will be understood that the components and other features outlined below can be applied to other nasal cannula (or tracheostomy mask) securement systems, including head gear, adhesive-based systems, or braces. The term "about," as used herein, should be understood to mean plus or minus 20%. For example, "about 20 mm" should be understood to mean 20 mm ± 4 mm.

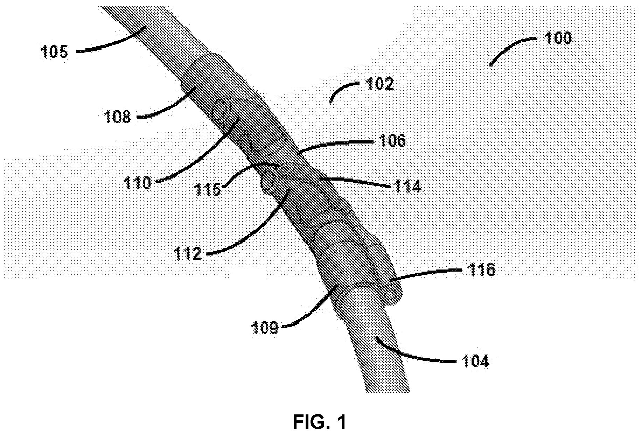

FIG. 1 shows a system 100 including a nasal cannula 102 and breathing gas supply tubes 104 and 105. Nasal cannula 102 includes a cannula body 106 having connectors 108 and 109 adapted to couple to breathing gas supply tubes 104 and 105, respectively, to receive flows of breathing gas. Nasal cannula 102 further includes nasal prongs 110 and 112 adapted to convey the flows of breathing gas into the nares of a patient. A sensing lumen 114 is disposed along nasal prong 112. Sensing lumen 114 includes a tip 115 and a housing 116 adapted to couple to a tube (not shown). Tip 115 may be disposed at a distal end of sensing lumen 114. As breathing gas is delivered to the patient via prongs 110 and 112, inhaled or exhaled gas flows past tip 115 or into sensing lumen 114 and changes the pressure within sensing lumen 114. A pressure sensor (not shown) may be disposed at a proximal end of sensing lumen 114 and detect the pressure changes within sensing lumen 114.

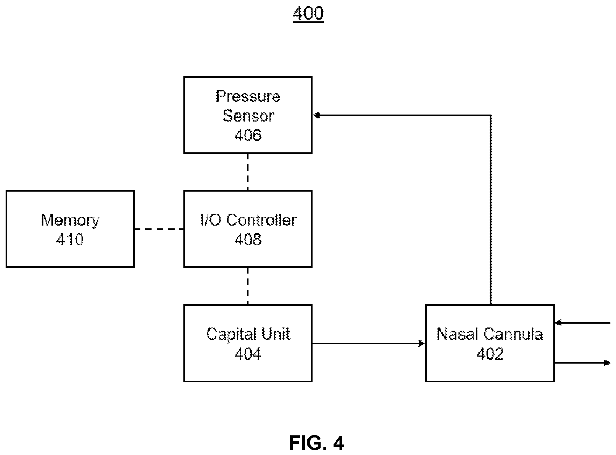

The pressure sensor is configured to output a pressure signal indicative of the detected pressure. By locating the pressure sensor away from cannula and, for example, at the end of the tube extending from the proximal end of sensing lumen 114, the pressure at the pressure sensor is conditioned to reduce noise in the signal. The pressure signal outputted by the pressure sensor may be communicated to a controller, for example, as described below in relation to FIG. 4. A pressure sensor capable of resolving pressure differences as small as about 1 Pa to about 4 Pa may be suitable. Such a high sensitivity pressure sensor allows detection of very small changes in pressure, for example, changes of about 1 Pa to about 4 Pa, or changes of less than 1 Pa. The pressure sensor may be configured to measure or determine absolute pressure within sensing lumen 114 or to measure or determine differential pressure relative to atmospheric pressure of the surrounding environment. In the latter implementation, the pressure sensor has a high resolution and includes an additional probe configured to measure the atmospheric pressure of the surrounding environment.

When the patient breathes, air flows past tip 115. Depending on whether the patient has their mouth open or closed and what the therapy flow rate is, the direction of flow past tip 115 may reverse during each breath or may only increase and decrease while maintaining the same direction. Either event causes pressure changes within sensing lumen 114, which may be detected or measured using the pressure sensor. The pressure may always be less than the environmental pressure (typically the case with open mouth breathing where the flow does not reverse) or may swing from positive to negative (typical with closed mouth breathing). When a patient stops breathing or removes cannula 102, the pressure changes stop and may be detected as an absence of pressure change.

In some implementations, cannula 102 includes a second sensing lumen having a second sensing tip positioned along the second nasal prong such that, during patient inspiration and expiration, gas flows over the second sensing tip to create a second pressure change within the second sensing lumen. In some implementations, cannula 102 includes a second pressure sensor configured to detect a second sensing lumen pressure in the second sensing lumen and positioned at a proximal end of the second sensing lumen opposite the second sensing tip. When two sensing lumens and two pressure sensors are used, the two detected pressures may be averaged to generate an average sensing lumen pressure. Alternatively, one of the two pressure sensors may be chosen, for example, using a controller as described herein and configured to receive a pressure signal from each pressure sensor, for example, if one nare becomes occluded such that inhaled or exhaled gas only or mostly flows over one of the two sensing tips.

Housing 116 is mounted on cannula body 106. For example, housing 116 may be integrally formed with cannula body 106. Alternatively, housing 116 may be detachably affixed to cannula body 106 via a clip-on connector, a magnet, a sliding lock, or another suitable form of attachment. A tube may be attached to the housing 116, for example at a proximal end of housing 116. The tube enables fluid communication between tip 115 and the pressure sensor. The tube and housing 116 define a fixed volume between tip 115 and the pressure sensor, and the pressure within this fixed volume changes as inhaled or exhaled gas flows toward tip 115 from the nare. The fixed volume is partially determined by the tube length and the tube inner diameter. Microbore tubing may be used such that the inner diameter is small, e.g., about 0.25 mm to about 2.5 mm. The small inner diameter minimizes the volume of air flow needed to conduct the pressure change along to the pressure sensor at the proximal end (of the tube). A flexible tube may be used in order to facilitate movement and repositioning of the pressure sensor while cannula 102 is in use. The tube may have a length between about 5 mm and about 3 m. As discussed above, the length of tube can serve to condition the pressure signal by reducing high frequency pressure fluctuations.

Tip 115 may be positioned on an exterior surface of prong 112. For example, tip 115 is positioned on a dorsal side of prong 112, for example, as shown in FIG. 1. For example, tip 115 may be positioned on a side of prong 112 farthest from the patient or on a top side of prong 112. Alternatively, tip 115 may be spaced from the tip of prong 112. Longitudinally, tip 115 may be flush with the tip of prong 112 or located proximally relative to the tip of prong 112, such that prong 112 extends farther into the nare than tip 115. In some implementations, tip 115 is set back from the tip of prong 112 such that tip 115 is outside of the nare while prong 112 extends at least partially into the nare.

Tip 115 may be angled relative to a longitudinal axis of sensing lumen 114. As noted previously, breath sensing with a high flow nasal cannula can be particularly difficult due to the high flow rates and open nature of the system, leading to high noise in the pressure signal. Constructing sensing lumen 114 with an angled tip 115 may reduce turbulence of the inhaled and/or exhaled gas, improving the pressure signal. For example, tip 115 may include a tip face at an angle other than 90 degrees relative to the longitudinal axis, such that the tip face is angled away from the outlet of prong 112. In some implementations, the angle is between about 30 degrees and about 60 degrees. In some implementations, the angle is between about 5 degrees and about 85 degrees. In some implementations, the angle is about 45 degrees. In some implementations, the angle is about 30 degrees or about 60 degrees. In some implementations, the angle is about 5 degrees or about 85 degrees. The tip face may have rounded, beveled, or chamfered edges, for example, to further reduce turbulence and prevent irritation or discomfort to the patient. Tip 115 may have an internal diameter between about 0.25 mm and about 2.5 mm. Tip 115 may have an internal diameter between about 0.10 mm and about 4.00 mm. In some implementations, tip 115 may have an internal diameter of about 0.25 mm or about 2.5 mm. In some implementations, tip 115 may have an internal diameter of about 0.5 mm, about 1.0 mm, about 1.5 mm, about 2.0 mm, or about 2.5 mm.

In some implementations, a control gas flows from a flow generator through at least a portion of the tube and/or sensing lumen 114. The control gas flows in a direction from the proximal end (e.g., of the tube) toward tip 115. In some implementations, the control gas flows in a direction from the proximal end of the sensing lumen 114 toward tip 115. The control gas may be introduced into the tube at a point near the pressure sensor, for example, via a tee joint in the tube that is fluidically connected to the flow generator. The control gas prevents liquids from entering sensing lumen 114 or the tube. The flow of control gas may be continuous or intermittent (e.g., as a pulse or single bolus of flow). A continuous flowrate of about 0.1 mL/min to about 10 mL/min can be effective to both prevent liquid ingress and maintain a stable, low pressure at the pressure sensor. The control gas may be supplied by a low pressure source (e.g., at <275 kPa, <200 kPa, <150 kPa, <100 kPa, <50 kPa, <30 kPa, <20 kPa, <10 kPa, <5 kPa or any other suitable gauge pressure). For an intermittent pulse or single bolus, a volume of about 1 mL to about 5 mL can clear liquid near tip 115 or elsewhere in sensing lumen 114. The control gas exits sensing lumen 114 at tip 115 and collides with inhaled or exhaled gas at a boundary region adjacent to tip 115, such that changes in pressure due to patient breathing are still detectable by the pressure sensor via sensing lumen 114. Suitable flow generators include a fan, a syringe pump, a blower, a compressor, a bellows, a wall air source, a pressurized tank, a piezo pump, or any other suitable source of pressurized gas. The flow generator may be incorporated in a respiratory system in use with cannula system 100 or may be separately configured with system 100 in a breath sensing device configured to adapt to various respiratory systems. For certain flowrates of control gas that lead to a high pressure at the pressure sensor, a pressure sensor with a larger pressure range may be used.

Suitable sources of pressurized breathing gas will be known to one of ordinary skill in the art. For example, the source may be the Vapotherm Flowrest System, Vapotherm HVT2.0 System, Precision Flow unit, or the Vapotherm 2000i, all of which are provided by Vapotherm, Inc. of Exeter, New Hampshire, USA. For example, the source may be the respiratory therapy systems described in U.S. Patent Application No. 16/901,902 (U.S. Publication No. 2021/0283357), and U.S. Patent Application No. 15/783,566 (U.S. Publication No. 2018/0104436). Other suitable sources of breathing gas will be known to one of ordinary skill in the art from the description herein, such as a blower, a compressor, a pressurized tank, a wall air outlet, or any other suitable gas source capable of generating high flow rates.

The source of breathing gas may be fluidically connected to first and second gas supply tubes 104 and 105 via a delivery tube (not shown) including an inlet configured to receive breathing gas from the source and a split outlet configured to divide and transmit breathing gas into the flows of breathing gas to the first and second gas supply tubes 104 and 105, respectively. The delivery tube may be insulated by an air or water jacket, or may be heated by an internal heated wire so as to maintain/regulate a temperature of the breathing gas provided to the patient. The tube providing the pressure to the pressure sensor is maintained separately from the delivery tube and external to any insulating means. In some implementations, the sensing lumen 114, tube, and/or pressure sensor are integrated into the delivery tube. For example, the tube conveying the pressure to the pressure sensor may be enclosed within a lumen of the delivery tube or embedded in a wall of the delivery tube, or the pressure sensor may be embedded in or affixed to the wall. The delivery tube may also serve to enclose any wires needed to connect the pressure sensor to a controller as described herein or the respiratory therapy system. The system 100 and delivery tube can be provided as a single unit to be attached to a variety of respiratory systems.

System 100 may be integrated in a respiratory therapy system having a breathing gas source and, optionally, a humidifying device (*e.g.,* a vapor transfer unit or hotpot humidifier). Alternatively, system 100 may be implemented as an add-on unit configured to couple to a compatible respiratory therapy system with a breathing gas source and, optionally, a humidifying device (*e.g.,* a vapor transfer unit or hotpot humidifier). System 100 may be used with a system that provides heated and humidified breathing gas. System 100 may be integrated into system 400 of FIG. 4 as nasal cannula 402, described below.

Suitable flowrates of breathing gas range from about 3 L/min to about 80 L/min. In some implementations, the flowrates of breathing gas is between about 20 L/min and about 70 L/min. In some implementations, the flowrates of breathing gas is between about 30 L/min and about 60 L/min. In some implementations, the breathing gas flowrate and nasal prong inner diameter are chosen such that the exit velocity of breathing gas from nasal prongs 110 and 112 is at a high velocity, for example, at least about 40 m/s. In some implementations, the exit velocity is between about 40 m/s and about 75 m/s for a balance effective flushing of CO₂ from the patient airway and patient comfort or noise reduction. In some implementations, an exit velocity between about 50 m/s and about 60 m/s is used. In some implementations, nasal prongs 110 and 112 have an internal diameter of about 1.0 mm to about 4.0 mm. In some implementations, nasal prongs 110 and 112 may have an internal diameter of about 1.5 mm to about 3.5 mm. In some implementations, nasal prongs 110 and 112 may have an internal diameter of about 2.0 mm to about 3.0 mm. For example, the ratio of sensing tip diameter to nasal prong diameter is between about 0.0625 and about 2.5.

In some implementations, a temperature sensor is used in addition to or in place of the pressure sensor. A temperature sensor can be applied in this context by measuring changes in temperature of the air near tip 115, because exhaled gas will have a higher temperature coming from within the patient's body than inhaled air from the atmosphere, presumed to be at or around room temperature, or possibly higher than room temperature due to heating of the delivered breathing gas. When using a temperature sensing in conjunction with the pressure sensor, the temperature sensing reading may be used to verify correlation between pressure signals and breathing patterns.

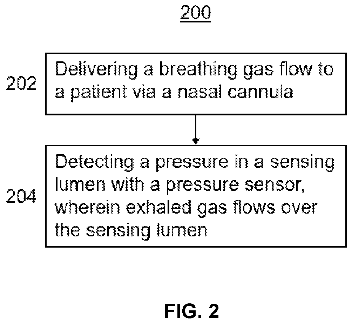

FIG. 2 shows a flowchart outlining an example method 200 for breath sensing while providing respiratory therapy to a patient. Method 200 includes steps 202 and 204. Step 202 involves delivering a flow of breathing gas to a nare of the patient via a nasal cannula. The nasal cannula includes a sensing lumen in fluid communication with a pressure sensor. Step 204 involves detecting a pressure within the sensing lumen of the nasal cannula using the pressure sensor. The sensing lumen is positioned such that gas inhaled or exhaled by the patient flows over a tip of the sensing lumen while the cannula is in use. The cannula may be cannula 102 described in relation to FIG. 1.

Method 200 may further include transmitting a signal indicative of the detected pressure from the pressure sensor to a controller, such as controller 408 of FIG. 4. The signal may indicate a detected pressure at a time point or a plurality of detected pressures over a period of time. The controller may be or may include a processor. The controller may be operatively coupled to a memory configured to store the signal, data based on the signal, and/or one or more algorithms for processing the signal. Method 200 may further include processing the signal using an algorithm stored in the memory. In some implementations, processing the signal includes determining a current breath rate or inspiratory/expiratory ratio of the patient. In some implementations, processing the signal includes identifying peaks in detected pressures over time. In some implementations, processing the signal includes determining an increase or decrease in pressure over time. In some implementations, processing the signal includes identifying an apnea event or a breathing pattern. In some implementations, processing the signal includes filtering, averaging, or smoothing the pressure data.

In some implementations, the algorithm is configured to determine an average pressure in the signal/data. The algorithm may determine maximum and minimum peaks in the data. The algorithm may determine the average maximum peak and/or the average minimum peak over a period of time. A pressure threshold may be determined as a percentage (e.g., between about 10% and about 30%) of the average maximum peak. This may allow the algorithm to identify a single breath of the patient based on when the pressure signal follows the following pattern: crosses the average pressure from below the average to above the average, then exceeds the average by at least the pressure threshold, then reaches a maximum peak, then cross the average from above the average to below the average, then reaches a minimum peak, and then crosses the average from below the average to above the average. The above steps may be based on a positive pressure swing, meaning that the pressure change is positive when the patient inhales. Alternatively, the pressure threshold may be determined as a percentage of the average minimum peak, and the algorithm steps are based on a negative pressure swing such that the pressure change is negative when the patient inhales. The pressure sensor used herein can be a relative pressure sensor, measuring the difference in pressure between two ports with one port positioned within the sensing lumen and one outside (measuring pressure of the surrounding environment), so the ports can be swapped to switch between the bases of positive and negative pressure swings.

Data representing information such as pressure signal, current breath rate, inspiratory/expiratory ratio, identified peaks, identified apnea events, or breathing patterns may be stored in the memory, for example, as an additional method step. The method may further include generating for display any of the above-listed information.

The above data and/or signal can be used to inform users, for example, clinicians, particularly as support for making clinical decisions related to respiratory therapy. For example, any of the above data can be displayed for informing a user or clinician. The data can also be transmitted to the gas source or another device to inform feedback-based control decisions. For example, information like breathing pattern or inspiratory/expiratory ratio determined from the pressure signal can be used to make adjustments to the provided therapy in a control loop or determine recommendations for therapy or treatment. Adjustments may include changing the breathing gas flowrate, breathing gas humidity, breathing gas temperature, oxygen percentage, oxygen flow rate, or aerosol concentration. These functionalities are described further in relation to FIG. 4 below.

In some implementations, the method includes conveying a control gas through the sensing lumen from a flow generator. As discussed above, the control gas prevents liquids from entering the sensing lumen. This feature is particularly helpful when the breathing gas is humidified, which leads to a higher likelihood of condensation buildup at the cannula. The flow of control gas may be continuous or intermittent (e.g., as a pulse). The control gas may be introduced into the tube at a point near the pressure sensor, for example, via a tee joint in the tube that is fluidically connected to the flow generator. A continuous flowrate of about 0.1 mL/min to about 10 mL/min can be effective to both prevent liquid ingress and maintain a stable, low pressure at the pressure sensor. In some implementations, a continuous flowrate can be between about 1.0 mL/min and about 7.5 mL/min, or between about 2.0 mL/min and 5 mL/min. The control gas may be supplied by a low pressure source (e.g., at <275 kPa, <200 kPa, <150 kPa, <100 kPa, <50 kPa, <30 kPa, <20 kPa, <10 kPa, <5 kPa or any other suitable gauge pressure). For an intermittent pulse, a volume of about 1 mL to about 5 mL can clear liquid near the tip of the sensing lumen. The control gas exits the sensing lumen at the tip and collides with inhaled or exhaled gas at a boundary region adjacent to the tip, such that changes in pressure due to patient breathing are still detectable by the pressure sensor via the sensing lumen. Suitable flow generators include a fan, a syringe pump, a blower, a compressor, a bellows, a wall air source, a pressurized tank, or any other suitable source of pressurized gas. The flow generator may be incorporated in a respiratory system in use with the cannula or may be separately configured with the in a breath sensing device configured to adapt to various respiratory systems. For certain flowrates of control gas that lead to a high pressure at the pressure sensor, a pressure sensor with a larger pressure range may be used.

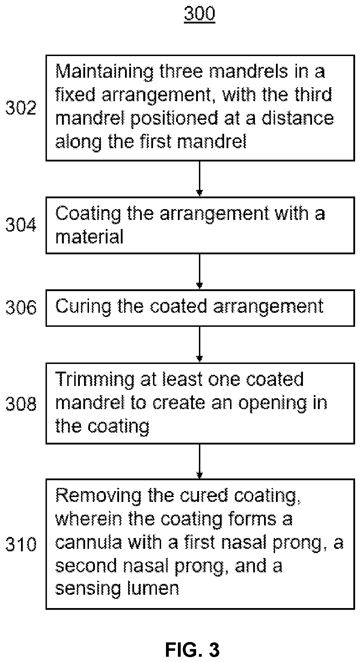

FIG. 3 shows a flowchart describing a method 300, not claimed, for manufacturing a nasal cannula for respiratory therapy and breath sensing. Method 300 includes steps 302, 304, 306, 308, and 310. Step 302 involves maintaining a first mandrel, a second mandrel, and a third mandrel in a fixed arrangement with respect to each other. In the fixed arrangement, the third mandrel is positioned along at least a portion of the first mandrel at a distance from the first mandrel. Step 304 involves coating the arrangement with a material. Step 306 involves curing the coated arrangement. Step 308 involves trimming at least one coated mandrel to create an opening in the coating of the trimmed mandrel. Step 310 involves removing the cured coating from the arrangement. The coating on the first, second, and third mandrels form a first nasal prong, a second nasal prong, and a sensing lumen, respectively, of the nasal cannula. For example, the cured coating may be a flexible plastic that is in the shape of the nosepiece shown in FIG. 1.

In some implementations, in step 304, coating the arrangement includes immersing the arrangement in the material and removing the arrangement from the material (e.g., dip molding). The relatively low monetary cost of the dip molding material allows for discarding the excess material without significantly affecting the production cost. In some implementations, in step 304, coating the arrangement includes spraying the material onto the arrangement. In an example, the first, second, and third mandrels are fixedly held on a substrate. This may keep the mandrels in the desired arrangement for the step of coating. The third mandrel may be held at a distance from the first mandrel in the arrangement. All of the first, second, and third mandrels may be fixed in the arrangement with a spacing between each other. By leaving a small distance between the mandrels, the material that flows around the mandrels will separate the structures formed by the mandrels with a thin layer of material.

For example, the coated arrangement may be cured at room temperature, or at a temperature or set of temperature (such as those determined by the material's curing temperature profile) determined to accelerate the curing time. For example, the coating may be cured using a heat lamp, oven, UV radiation, or any other suitable means. After curing, at least one coated mandrel may be trimmed to create an opening in the coating of the trimmed mandrel.

The mandrels may be made of, for example, steel, aluminum-bronze alloys, stainless steel, or any other suitable material, such as those resistance to curing methods and which will not permanently adhere to the flexible material used in forming the nosepiece. The materials used to manufacture the cannula in general may also be selected so as to facilitate smooth flow within the tubing and appropriate durability and connectivity on the outside of the tubing. In some implementations a material of the outer surface includes at least one of: polyvinyl chloride (PVC) plastic, plastisol, vinyl, silicone, non-latex rubber, an elastomer, ethylene vinyl acetate (EVA), styrene-butadiene copolymer (SBC), polyether ether ketone (PEEK), a polyether block amide (such as PEBAX), a polyethylene material, a high-density polyethylene (HDPE) material, a medium-density polyethylene (MDPE) material, a low-density polyethylene (LDPE) material, a crack-resistant material, a material with a low coefficient of friction, a material less than 70 Durometer Shore A, and flexible plastic. Flexible plastics and the other material examples listed above may be chosen to provide customized comfort to a patient. For example, an infant patient may require a more flexible nosepiece than an adult patient. In such an example, a more flexible material may be chosen.

FIG. 4 shows a block diagram of a system 400 including nasal cannula 402, capital unit 404, pressure sensor 406, controller 408, and memory 410. Capital unit 404 includes a pressurized breathing gas source (not shown) configured to deliver a flow of breathing gas to nasal cannula 402. Nasal cannula 402 is configured to convey the flow of breathing gas at a high velocity (e.g., at least at about 40 m/s) into the nares of a patient. In some implementations, the exit velocity is between about 40 m/s and about 75 m/s. In some implementations, an exit velocity between about 50 m/s and about 60 m/s is used. As the patient breathes, a sensing lumen of nasal cannula 402 conveys pressure changes to pressure sensor 406, wherein the pressure changes result from patient inspiration and expiration. Pressure sensor 406 is configured to detect the pressure changes and output a signal indicative of the detected pressure to controller 408. Controller 408 is operatively coupled to pressure sensor 406 and memory 410. Controller 408 is configured to receive and process the signal from pressure sensor 406. Memory 410 is configured to store information such as the received signal and an algorithm for signal processing. Controller 408 is optionally operatively coupled to capital unit 404. In an example, nasal cannula 402 is cannula 102 of FIG. 1.

In some implementations, processing the signal includes determining a current breath rate or inspiratory/expiratory ratio of the patient. In some implementations, processing the signal includes identifying peaks in detected pressures over time. In some implementations, processing the signal includes determining an increase or decrease in pressure over time. In some implementations, processing the signal includes identifying an apnea event or a breathing pattern. In some implementations, processing the signal includes applying a filter to the signal to remove noise. In some implementations, processing the signal includes identifying and excluding non-breathing periods (e.g., swallowing, sneezing, talking, or coughing) from the data. By ignoring non-breathing artifacts, controller 408 may more accurately calculate breathing parameters.

Processing the signal involves determining an average pressure in the signal/data. Maximum peaks, minimum peaks, the average maximum peak over a period of time, and the average minimum peak over a period of time are also determined. A pressure threshold is determined as a percentage (e.g., between about 10% and about 30%) of the average maximum peak. This allows controller 408 to identify a single breath of the patient based on when the pressure signal follows the following pattern: crosses the average pressure from below the average to above the average, then exceeds the average by at least the pressure threshold, then reaches a maximum peak, then cross the average from above the average to below the average, then reaches a minimum peak, and then crosses the average from below the average to above the average. The above steps may be based on a positive pressure swing, meaning that the pressure change is positive when the patient inhales. Alternatively, the pressure threshold may be determined as a percentage of the average minimum peak, and the algorithm steps are based on a negative pressure swing such that the pressure change is negative when the patient inhales. The pressure sensor used herein can be a relative pressure sensor, measuring the difference in pressure between two ports with one port positioned within the sensing lumen and one outside (measuring pressure of the surrounding environment), so the ports can be swapped to switch between the bases of positive and negative pressure swings.

In some cases, the patient's breathing pattern may change, resulting in breathing that does not reach the threshold for breath detection that has been established from previous average peaks. This can occur if the patient switches to shallow breathing after a period of deep breathing or when a patient switches from closed-mouth to open-mouth breathing. In these cases, an algorithm can detect that there is sufficient pressure change (e.g., one pressure change or multiple pressure changes) to indicate that breathing is occurring even though it is not reaching the minimum threshold of breath detection. The averages are reset and new average peaks are determined from the pressure data.

The above-identified signal processing, with or without any of the optional processing steps discussed above, is executed by an algorithm stored in memory 410. Data representing information such as pressure signal, current breath rate, inspiratory/expiratory ratio, identified peaks, identified apnea events, or breathing patterns may be stored in memory 410. System 400 may further include a screen or display configured to display any of the above information.

When controller 408 is operatively coupled to capital unit 404, controller 408 may be configured to transmit information or data to capital unit 404. For example, controller 408 is configured to notify capital unit 404 of a non-breathing period or apnea event, and in response capital unit 404 may vary the flow of breathing gas and/or generates an alarm. Controller 408 may transmit a pressure signal, current breath rate, inspiratory/expiratory ratio, identified peaks, identified apnea events, or breathing patterns to capital unit 404. Capital unit 404, in response to receiving any of the above information, may adjust one or more operational parameters. For example, capital unit 404 increases or decreases one or more of breathing gas flow rate, breathing gas humidity, breathing gas temperature, oxygen percentage, oxygen flow rate, or aerosol concentration. This feature may allow a caretaker to wean the user off of respiratory therapy when they demonstrate improved breathing. Capital unit 404, in response to receiving any of the above information, may generate an alarm to notify a user or caretaker. This feature may be beneficial for compliance monitoring.

Capital unit 404 includes a pressurized breathing gas source that may be one of a blower, a compressor, a pressurized tank, a wall air outlet, or any other suitable gas source capable of generating high flow rates (e.g., between about 1 L/min and 60 L/min, or greater). In some implementations, capital unit 404 is configured to output a heated and humidified flow of breathing gas. For example, capital unit 404 contains a vapor transfer unit or hotpot humidifier configured to evaporate water into the flow of breathing gas. In some implementations, capital unit 404 includes a port for oxygen titration. For example, an oxygen tank, oxygen concentrator, or oxygen wall outlet is coupled to capital unit 404, and a flow of oxygen is added to the breathing gas to reach a target oxygen concentration or oxygen flow rate. In some implementations, an aerosolized medicament is entrained in the flow of breathing gas and delivered simultaneously to the patient. For example, a nebulizer is mounted to nasal cannula 402, to capital unit 404, or to a disposable unit on capital unit 404, and the nebulizer emits aerosol medicament particles into the flow of breathing gas.

In some implementations, capital unit 402, nasal cannula 404, pressure sensor 406, controller 408, and memory 410 are parts of one system 400. In other implementations, capital unit 404 is a respiratory system, and a breath sensing device separately houses pressure sensor 406, controller 408, and memory 410, and is adapted to couple to capital unit 404 and nasal cannula 402. The breath sensing device may be packaged and adapted to couple to existing respiratory therapy systems having a suitable pressurized breathing gas source. In some implementations the system 400 includes additional medical monitoring devices such as a pulse oximeter device or transcutaneous carbon dioxide sensor, and the breath sensing data detected by the pressure sensor 406 is displayed at the capital unit 402 with additional parameters detected at the other monitoring devices.

The foregoing is merely illustrative of the principles of the disclosure, and the apparatuses can be practiced by other than the described implementations, which are presented for purposes of illustration and not of limitation. It is to be understood that the apparatuses disclosed herein, while shown for use in high flow therapy systems, may be applied to systems to be used in other ventilation circuits.

Variations and modifications will occur to those of skill in the art after reviewing this disclosure. The disclosed features may be implemented, in any combination and subcombination (including multiple dependent combinations and subcombinations), with one or more other features described herein. The various features described or illustrated above, including any components thereof, may be combined or integrated in other systems. Moreover, certain features may be omitted or not implemented.

Examples of changes, substitutions, and alterations are ascertainable by one skilled in the art and could be made without departing from the scope of the claims.

## Claims

1. A breath sensing device (100) configured to monitor patient breathing, the device comprising:
a nasal cannula (102,402) for breath sensing of a patient being provided high flow respiratory therapy, the nasal cannula comprising:
a cannula body (106) having a first connector (108) configured to receive a first flow of breathing gas and a second connector (109) configured to receive a second flow of breathing gas;
a first nasal prong (110) configured to deliver the first flow of breathing gas to a nare of the patient;
a second nasal prong (112) configured to deliver the second flow of breathing gas to a nare of the patient;
a first sensing lumen (114) having a first sensing tip (115) positioned along the first nasal prong such that, during patient inspiration and expiration, gas flows over the first sensing tip to create a pressure change within the first sensing lumen; and
a first pressure sensor (406) configured to detect a first pressure in the first sensing lumen, the first pressure sensor being positioned at a proximal end of the first sensing lumen opposite the first sensing tip; and
a controller (408) configured to:
receive a pressure signal from the first pressure sensor indicative of the first sensing lumen pressure;
convert the pressure signal into data; and
apply an algorithm to the data,
**characterized in that**, the algorithm is configured to:
determine an average pressure in the data;
determine maximum and minimum peaks in the data and/or filter the data;
determine an average maximum peak over a period of time;
determine an average minimum peak over a period of time;
determine a pressure threshold as a percentage of the average maximum peak; and
identify a breath when the pressure:
crosses from below the average pressure to above the average pressure, then
exceeds the average pressure by at least the pressure threshold, then
reaches a maximum peak, then
crosses from above the average pressure to below the average pressure, then
reaches a minimum peak, then
crosses from below the average pressure to above the average pressure.

2. The breath sensing device of claim 1, wherein the first sensing lumen comprises:
a housing (116) mounted on the cannula body, and
a tube configured to enable fluid communication between the first sensing tip and the pressure sensor, wherein the tube is in fluid communication with the pressure sensor at the proximal end of the first sensing lumen,
wherein the housing is fluidically connected to the tube.

3. The breath sensing device of claim 2, wherein:
the tube has a length between about 5 mm and about 3 m; and/or
the tube has an internal diameter between about 0.25 mm and about 2.5 mm.

4. The breath sensing device of claim 3, configured for flow of a control gas from a flow generator through at least a portion of the tube in a direction from the proximal end to the sensing tip, wherein at least one of:
the nasal cannula is configured for collision of the control gas with patient breath at a boundary region adjacent to the first sensing tip;
the device is configured to deliver the control gas intermittently as a bolus flow;
the device is configured to deliver the control gas at a flow rate between about 0.1 mL/min and about 10 mL/min;
the flow generator is one of a fan, a syringe pump, a blower, a compressor, a bellows, or a wall air source;
the nasal cannula is configured with the control gas so as to purge condensation from the first sensing lumen; and
the sensing lumen comprises a tee joint positioned along the tube and configured to convey the control gas into the tube from the flow generator.

5. The breath sensing device of any of the preceding claims, wherein at least one of:
the nasal cannula further comprises a temperature sensor configured to measure a breath temperature;
the detected first pressure indicates a difference between an internal pressure within the first sensing lumen and ambient pressure of the surrounding environment;
the first pressure sensor is configured to output a pressure signal indicative of the detected first sensing lumen pressure;
the first sensing tip is angled relative to a longitudinal axis of the first sensing lumen;
the sensing tip is positioned on an exterior surface of the first nasal prong;
the sensing tip is positioned proximally to a prong tip of the first nasal prong;
the sensing tip has an internal diameter between about 0.25 mm and 2.5 mm; and
the first nasal prong has an internal diameter of about 1.0 to about 4.0 mm.

6. The breath sensing device of any of the preceding claims, wherein the first sensing tip comprises a tip face at an angle other than 90 degrees relative to a longitudinal axis of the first sensing lumen, and wherein:
the angle is between 30 degrees and 60 degrees; and/or
the tip face has rounded, beveled, or chamfered edges.

7. The breath sensing device of any of the preceding claims, wherein the algorithm is configured to identify and exclude non-breathing periods from the data.

8. The breath sensing device of any of the preceding claims, wherein the nasal cannula further comprises:
a second sensing lumen having a second sensing tip positioned along the second nasal prong such that, during patient inspiration and expiration, gas flows over the second sensing tip to create a second pressure change within the second sensing lumen; and
a second pressure sensor configured to detect a second pressure in the second sensing lumen and positioned at a proximal end of the second sensing lumen opposite the second sensing tip.

9. The breath sensing device of claim 8, wherein the controller is configured to average data from the first pressure sensor and the second pressure sensor to generate an average sensing lumen pressure.

10. The breath sensing device of claim 9, wherein the controller is configured to receive data from only one of the first pressure sensor or the second pressure sensor when one of the nares of the patient is blocked.

11. Apparatus comprising:
a respiratory therapy system; and
the breath sensing device of any of the preceding claims,
wherein the device is configured to couple to the respiratory therapy system.

12. The apparatus of claim 11, wherein:
the controller is configured to either send instructions to the respiratory therapy system to change an operating parameter of the respiratory therapy system, or to send the data to the respiratory therapy system for determining a change to an operating parameter of the respiratory therapy system; and/or the respiratory therapy system is configured to provide the first flow of breathing gas to the nasal cannula.

13. The apparatus of claim 11 or 12, wherein at least one of:
the controller is configured to determine periods of patient inspiration and/or expiration based on the data from the first pressure sensor;
the controller is configured to determine and display at least one of patient expiratory/inspiratory ratio, a current breath rate, or a patient breathing pattern based on the data from the first pressure sensor.

14. A method for sensing patient breathing when the patient is provided with high flow respiratory therapy, the method comprising:
detecting a pressure in a sensing lumen of a nasal cannula using a pressure sensor positioned at a proximal end of the sensing lumen opposite a tip of the sensing lumen, wherein the sensing lumen is positioned such that, during patient inspiration and expiration, gas flows over the tip of the sensing lumen to create a pressure change within the sensing lumen;
transmitting from the pressure sensor to a controller a signal indicative of a plurality of pressures detected by the pressure sensor over a period of time;
processing the signal using an algorithm stored in a memory of the controller, **characterized in that**, processing the signal comprises identifying peaks in the plurality of detected pressures over time and determining a current breath rate;
displaying the current breath rate;
determining an average pressure in the data;
determining maximum and minimum peaks in the data and/or filtering the data;
determining an average maximum peak over a period of time;
determining an average minimum peak over a period of time;
determining a pressure threshold as a percentage of the average maximum peak; and
determining a breath when the pressure:
crosses from below the average pressure to above the average pressure, then exceeds the average pressure by at least the pressure threshold reaches a maximum peak, then
crosses from above the average pressure to below the average pressure, then reaches a minimum peak, then
crosses from below the average pressure to above the average pressure.

15. The method of claim 14, wherein the detected pressure is a differential pressure between a pressure within the sensing lumen and an ambient pressure.

## Patentansprüche

1. Atemsensorgerät (100), das dazu konfiguriert ist, die Atmung eines Patienten zu überwachen, wobei das Gerät eine Nasenkanüle (102, 402) zur Atemerfassung eines Patienten umfasst, dem eine Hochfluss-Atemtherapie verabreicht wird, wobei die Nasenkanüle Folgendes umfasst:
einen Kanülenkörper (106) mit einem ersten Anschlussstück (108), das zur Aufnahme eines ersten Atemgasstroms konfiguriert ist, und einem zweiten Anschlussstück (109), das zur Aufnahme eines zweiten Atemgasstroms konfiguriert ist;
einen ersten Nasenstecker (110), der zur Abgabe des ersten Atemgasstroms an ein Nasenloch des Patienten konfiguriert ist;
einen zweiten Nasenstecker (112), der zur Abgabe des zweiten Atemgasstroms an ein Nasenloch des Patienten konfiguriert ist;
ein erstes Sensorlumen (114) mit einer ersten Sensorspitze (115), die entlang des ersten Nasensteckers positioniert ist, so dass während des Ein- und Ausatmens des Patienten Gas über die erste Sensorspitze strömt, um eine Druckänderung innerhalb des ersten Sensorlumens zu erzeugen; und
einen ersten Drucksensor (406), der dazu konfiguriert ist, einen ersten Druck im ersten Sensorlumen zu erfassen, wobei der erste Drucksensor an einem proximalen Ende des ersten Sensorlumens gegenüber der ersten Sensorspitze positioniert ist; und
eine Steuerung (408), die dazu konfiguriert ist:
ein Drucksignal vom ersten Drucksensor zu empfangen, das den ersten Sensorlumendruck angibt;
das Drucksignal in Daten umzuwandeln; und
einen Algorithmus auf die Daten anzuwenden,
**dadurch gekennzeichnet, dass** der Algorithmus dazu konfiguriert ist:
einen durchschnittlichen Druck in einem zu bestimmen:
maximale und minimale Spitzen in den Daten zu bestimmen und/oder die Daten zu filtern;
einen durchschnittlichen maximalen Spitzenwert über einen Zeitraum zu bestimmen;
einen durchschnittlichen minimalen Spitzenwert über einen Zeitraum zu bestimmen;
einen Druckschwellenwert als Prozentsatz des durchschnittlichen Maximalwerts zu bestimmen; und
einen Atemzug zu identifizieren, wenn der Druck:
von unterhalb des Durchschnittsdrucks auf oberhalb des Durchschnittsdrucks wechselt, dann
den Durchschnittsdruck um mindestens den Druckschwellenwert überschreitet, dann einen Maximalwert erreicht, dann
von oberhalb des Durchschnittsdrucks auf unterhalb des Durchschnittsdrucks wechselt, dann
einen Minimalwert erreicht, dann
von unterhalb des Durchschnittsdrucks auf oberhalb des Durchschnittsdrucks wechselt.

2. Atemsensorgerät nach Anspruch 1, wobei das erste Sensorlumen Folgendes umfasst:
ein Gehäuse (116), das auf dem Kanülenkörper montiert ist, und
ein Rohr, das so konfiguriert ist, dass es eine Flüssigkeitsverbindung zwischen der ersten Sensorspitze und dem Drucksensor ermöglicht, wobei das Rohr in Flüssigkeitsverbindung mit dem Drucksensor am proximalen Ende des ersten Sensorlumens steht,
wobei das Gehäuse flüssigkeitsmäßig mit dem Rohr verbunden ist.

3. Atemsensorgerät nach Anspruch 2, wobei:
das Rohr eine Länge zwischen etwa 5 mm und etwa 3 m aufweist; und/oder
das Rohr einen Innendurchmesser zwischen etwa 0,25 mm und etwa 2,5 mm aufweist.

4. Atemsensorgerät nach Anspruch 3, das für den Fluss eines Steuergases von einem Flussgenerator durch mindestens einen Teil des Rohrs in einer Richtung vom proximalen Ende zur Sensorspitze konfiguriert ist, wobei mindestens:
die Nasenkanüle für eine Kollision des Steuergases mit dem Atem des Patienten an einem Grenzbereich neben der ersten Sensorspitze konfiguriert ist;
das Gerät so konfiguriert ist, dass es das Steuergas intermittierend als Bolusfluss abgibt;
das Gerät so konfiguriert ist, dass es das Steuergas mit einer Flussrate zwischen etwa 0,1 ml/min und etwa 10 ml/min abgibt;
der Flussgenerator eines von einem Ventilator, einer Spritzenpumpe, einem Gebläse, einem Kompressor, einem Blasebalg oder einer Wandluftquelle ist;
die Nasenkanüle mit dem Steuergas konfiguriert ist, um Kondensation aus dem ersten Sensorlumen zu entfernen; und
das Sensorlumen eine T-Verbindung umfasst, die entlang des Rohrs positioniert ist und so konfiguriert ist, dass sie das Steuergas vom Flussgenerator in das Rohr leitet.

5. Atemsensorgerät nach einem der vorhergehenden Ansprüche, wobei mindestens:
die Nasenkanüle außerdem einen Temperatursensor umfasst, der zum Messen einer Atemtemperatur konfiguriert ist;
der erfasste erste Druck eine Differenz zwischen einem Innendruck innerhalb des ersten Sensorlumens und dem Umgebungsdruck der umgebenden Umwelt anzeigt;
der erste Drucksensor zum Ausgeben eines Drucksignals konfiguriert ist, das den erfassten Druck im ersten Sensorlumen angibt;
die erste Sensorspitze relativ zu einer Längsachse des ersten Sensorlumens abgewinkelt ist;
die Sensorspitze auf einer Außenfläche des ersten Nasensteckers positioniert ist;
die Sensorspitze proximal zu einer Steckerspitze des ersten Nasensteckers positioniert ist;
die Sensorspitze einen Innendurchmesser zwischen etwa 0,25 mm und 2,5 mm aufweist; und
der erste Nasenstecker einen Innendurchmesser von etwa 1,0 bis etwa 4,0 mm aufweist.

6. Atemsensorgerät nach einem der vorhergehenden Ansprüche, wobei die erste Sensorspitze eine Spitzenfläche in einem Winkel ungleich 90 Grad relativ zu einer Längsachse des ersten Sensorlumens umfasst, und wobei:
der Winkel zwischen 30 Grad und 60 Grad liegt; und/oder die Spitzenfläche abgerundete, abgeschrägte oder abgefaste Kanten aufweist.

7. Atemsensorgerät nach einem der vorhergehenden Ansprüche, wobei der Algorithmus so konfiguriert ist, dass er Nicht-Atemperioden aus den Daten identifiziert und ausschließt.

8. Atemsensorgerät nach einem der vorhergehenden Ansprüche, wobei die Nasenkanüle ferner Folgendes umfasst:
ein zweites Sensorlumen mit einer zweiten Sensorspitze, die entlang des zweiten Nasensteckers positioniert ist, so dass während des Ein- und Ausatmens des Patienten Gas über die zweite Sensorspitze strömt, um eine zweite Druckänderung innerhalb des zweiten Sensorlumens zu erzeugen; und
einen zweiten Drucksensor, der so konfiguriert ist, dass er einen zweiten Druck im zweiten Sensorlumen erkennt und an einem proximalen Ende des zweiten Sensorlumens gegenüber der zweiten Sensorspitze positioniert ist.

9. Atemsensorgerät nach Anspruch 8, wobei die Steuerung so konfiguriert ist, dass sie Daten vom ersten Drucksensor und vom zweiten Drucksensor mittelt, um einen durchschnittlichen Lumendruck zu erzeugen.

10. Atemsensorgerät nach Anspruch 9, wobei die Steuerung so konfiguriert ist, dass sie Daten nur vom ersten Drucksensor oder vom zweiten Drucksensor empfängt, wenn eines der Nasenlöcher des Patienten blockiert ist.

11. Vorrichtung, umfassend:
ein Atemtherapiesystem; und
das Atemsensorgerät nach einem der vorhergehenden Ansprüche,
wobei das Gerät so konfiguriert ist, dass es mit dem Atemtherapiesystem gekoppelt werden kann.

12. Vorrichtung nach Anspruch 11, wobei:
die Steuerung so konfiguriert ist, dass sie entweder Anweisungen zum Ändern eines Betriebsparameters des Atemtherapiesystems an das Atemtherapiesystem sendet oder die Daten zum Bestimmen einer Änderung eines Betriebsparameters des Atemtherapiesystems an das Atemtherapiesystem sendet; und/oder das Atemtherapiesystem so konfiguriert ist, dass es den ersten Atemgasfluss an die Nasenkanüle liefert.

13. Vorrichtung nach Anspruch 11 oder 12, wobei mindestens:
die Steuerung dazu konfiguriert ist, dass sie anhand der Daten des ersten Drucksensors Einatem- und/oder Ausatemperioden des Patienten bestimmt;
die Steuerung so konfiguriert ist, dass sie anhand der Daten des ersten Drucksensors mindestens eines von Einatem-/Ausatemverhältnis des Patienten, aktueller Atemfrequenz oder Atemmuster des Patienten bestimmt und anzeigt.

14. Verfahren zum Erfassen der Atmung eines Patienten, wenn der Patient einer Hochfluss-Atemtherapie unterzogen wird, wobei das Verfahren Folgendes umfasst:
Erfassen eines Drucks in einem Messlumen einer Nasenkanüle mithilfe eines Drucksensors, der an einem proximalen Ende des Messlumens gegenüber einer Spitze des Messlumens positioniert ist, wobei das Messlumen so positioniert ist, dass während des Ein- und Ausatmens des Patienten Gas über die Spitze des Messlumens strömt, um eine Druckänderung innerhalb des Messlumens zu erzeugen;
Übertragen eines Signals vom Drucksensor an eine Steuerung, das eine Vielzahl von Drücken anzeigt, die vom Drucksensor über einen Zeitraum erfasst wurden;
Verarbeiten des Signals mithilfe eines Algorithmus, der in einem Speicher der Steuerung gespeichert ist,
**dadurch gekennzeichnet, dass**
das Verarbeiten des Signals Folgendes umfasst Identifizieren von Spitzen in der Vielzahl der erfassten Drücke im Zeitverlauf und das Bestimmen einer aktuellen Atemfrequenz;
Anzeigen der aktuellen Atemfrequenz;
Bestimmen eines Durchschnittsdrucks in den Daten;
Bestimmen von maximalen und minimalen Spitzen in den Daten und/oder das Filtern der Daten;
Bestimmen einer durchschnittlichen maximalen Spitze über einen Zeitraum;
Bestimmen einer durchschnittlichen minimalen Spitze über einen Zeitraum;
Bestimmen einer Druckschwelle als Prozentsatz der durchschnittlichen maximalen Spitze; und
Bestimmen eines Atemzugs, wenn der Druck:
von unterhalb des Durchschnittsdrucks auf oberhalb des Durchschnittsdrucks wechselt, dann
den Durchschnittsdruck um mindestens die Druckschwelle überschreitet, eine maximale Spitze erreicht, dann
von oberhalb des Durchschnittsdrucks auf unterhalb des Durchschnittsdrucks wechselt, dann
eine minimale Spitze erreicht, dann
von unterhalb des Durchschnittsdrucks auf oberhalb des Durchschnittsdrucks wechselt.

15. Verfahren nach Anspruch 14, wobei der erfasste Druck ein Differenzdruck zwischen einem Druck innerhalb des Sensorlumens und einem Umgebungsdruck ist.

## Revendications

1. Dispositif de détection de respiration (100) conçu pour surveiller la respiration d'un patient, le dispositif comprenant une canule nasale (102,402) pour détecter la respiration d'un patient recevant une thérapie respiratoire à haut débit, la canule nasale comprenant :
un corps de canule (106) comportant un premier connecteur (108) conçu pour recevoir un premier flux de gaz respiratoire et un second connecteur (109) conçu pour recevoir un second flux de gaz respiratoire ;
une première broche nasale (110) conçue pour délivrer le premier flux de gaz respiratoire à une narine du patient ;
une seconde broche nasale (112) conçue pour délivrer le second flux de gaz respiratoire à une narine du patient ;
une première lumière de détection (114) comportant une première pointe de détection (115) positionnée le long de la première broche nasale de telle sorte que, pendant l'inspiration et l'expiration du patient, le gaz s'écoule sur la première pointe de détection pour créer un changement de pression dans la première lumière de détection ; et
un premier capteur de pression (406) conçu pour détecter une première pression dans la première lumière de détection, le premier capteur de pression étant positionné à une extrémité proximale de la première lumière de détection opposée à la première pointe de détection ; et
un dispositif de commande (408) conçu pour :
recevoir un signal de pression provenant du premier capteur de pression indiquant la première pression de la lumière de détection ;
convertir le signal de pression en données ; et
appliquer un algorithme aux données,
**caractérisé en ce que** l'algorithme est conçu pour :
déterminer une pression moyenne dans les données ;
déterminer les pics maximum et minimum dans les données et/ou filtrer les données ;
déterminer un pic maximum moyen sur une période donnée ;
déterminer un pic minimum moyen sur une période donnée ;
déterminer un seuil de pression en pourcentage du pic maximum moyen ;et
identifier une respiration lorsque la pression :
passe d'au-dessous de la pression moyenne à au-dessus de la pression moyenne, puis
dépasse la pression moyenne d'au moins le seuil de pression, puis
atteint un pic maximum, puis
passe d'au-dessus de la pression moyenne à au-dessous de la pression moyenne, puis
atteint un pic minimum, puis
passe d'au-dessous de la pression moyenne à au-dessus de la pression moyenne.

2. Dispositif de détection de respiration selon la revendication 1, dans lequel la première lumière de détection comprend :
un boîtier (116) monté sur le corps de la canule, et
un tube conçu pour permettre une communication fluidique entre la première pointe de détection et le capteur de pression, le tube étant en communication fluidique avec le capteur de pression à l'extrémité proximale de la première lumière de détection,
dans lequel le boîtier est relié fluidiquement au tube.

3. Dispositif de détection de respiration selon la revendication 2, dans lequel :
le tube a une longueur comprise entre environ 5 mm et 3 m ; et/ou
le tube a un diamètre interne compris entre environ 0,25 mm et environ 2,5 mm.

4. Dispositif de détection de respiration selon la revendication 3, conçu pour l'écoulement d'un gaz de commande à partir d'un générateur d'écoulement à travers au moins une partie du tube dans une direction allant de l'extrémité proximale à la pointe de détection, dans lequel au moins l'un des éléments suivants :
la canule nasale est conçue pour la collision du gaz de commande avec la respiration du patient dans une région limite adjacente à la première pointe de détection ;
le dispositif est conçu pour délivrer le gaz de commande par intermittence sous forme d'écoulement du bolus ;
le dispositif est conçu pour délivrer le gaz de commande à un débit compris entre environ 0,1 ml/min et environ 10 ml/min ;
le générateur de flux est un ventilateur, une pompe à seringue, une soufflerie, un compresseur, un soufflet ou une source d'air murale ;
la canule nasale est conçue avec le gaz de commande de manière à purger la condensation de la première lumière de détection ; et
la lumière de détection comprend un joint en T positionné le long du tube et conçu pour transporter le gaz de commande dans le tube à partir du générateur de flux.

5. Dispositif de détection de respiration selon l'une quelconque des revendications précédentes, dans lequel au moins l'un des éléments suivants :
la canule nasale comprend en outre un capteur de température conçu pour mesurer une température de respiration ;
la première pression détectée indique une différence entre une pression interne dans la première lumière de détection et la pression ambiante de l'environnement ;
le premier capteur de pression est conçu pour émettre un signal de pression indiquant la première pression de lumière de détection détectée ;
la première pointe de détection est inclinée par rapport à un axe longitudinal de la première lumière de détection ;
la pointe de détection est positionnée sur une surface extérieure de la première broche nasale ;
la pointe de détection est positionnée de manière proximale par rapport à une pointe de broche de la première broche nasale ;
la pointe de détection a un diamètre interne compris entre environ 0,25 mm et 2,5 mm ; et
la première broche nasale a un diamètre interne d'environ 1,0 à environ 4,0 mm.

6. Dispositif de détection de respiration selon l'une quelconque des revendications précédentes, dans lequel la première pointe de détection comprend une face de pointe à un angle différent de 90 degrés par rapport à un axe longitudinal de la première lumière de détection, et
dans lequel :
l'angle est compris entre 30 degrés et 60 degrés ; et/ou
la face de la pointe présente des bords arrondis, biseautés ou chanfreinés.

7. Dispositif de détection de respiration selon l'une quelconque des revendications précédentes, dans lequel l'algorithme est conçu pour identifier et exclure des données les périodes de non-respiration.

8. Dispositif de détection de respiration selon l'une quelconque des revendications précédentes, dans lequel la canule nasale comprend en outre :
une seconde lumière de détection comportant une seconde pointe de détection positionnée le long de la seconde broche nasale de telle sorte que, pendant l'inspiration et l'expiration du patient, le gaz s'écoule sur la seconde pointe de détection pour créer un second changement de pression dans la seconde lumière de détection ; et
un second capteur de pression conçu pour détecter une seconde pression dans la seconde lumière de détection et positionné à une extrémité proximale de la seconde lumière de détection opposée à la seconde pointe de détection.

9. Dispositif de détection de respiration selon la revendication 8, dans lequel le dispositif de commande est conçu pour faire la moyenne des données provenant du premier capteur de pression et du second capteur de pression afin de générer une pression de lumière de détection moyenne.

10. Dispositif de détection de respiration selon la revendication 9, dans lequel le dispositif de commande est conçu pour recevoir des données provenant uniquement de l'un du premier capteur de pression ou du second capteur de pression lorsque l'une des narines du patient est bloquée.

11. Appareil comprenant :
un système de thérapie respiratoire ; et
le dispositif de détection de respiration selon l'une quelconque des revendications précédentes,
dans lequel le dispositif est conçu pour se coupler au système de thérapie respiratoire.

12. Appareil selon la revendication 11, dans lequel :
le dispositif de commande est conçu pour envoyer soit des instructions au système de thérapie respiratoire pour modifier un paramètre de fonctionnement du système de thérapie respiratoire, soit pour envoyer les données au système de thérapie respiratoire pour déterminer une modification d'un paramètre de fonctionnement du système de thérapie respiratoire ; et/ou le système de thérapie respiratoire est conçu pour fournir le premier flux de gaz respiratoire à la canule nasale.

13. Appareil selon la revendication 11 ou 12, dans lequel au moins l'un des éléments suivants :
le dispositif de commande est conçu pour déterminer les périodes d'inspiration et/ou d'expiration du patient sur la base des données provenant du premier capteur de pression ;
le dispositif de commande est conçu pour déterminer et afficher au moins l'un des éléments suivants le rapport expiratoire/inspiratoire du patient, une fréquence respiratoire actuelle ou un modèle de respiration du patient sur la base des données provenant du premier capteur de pression.

14. Procédé de détection de la respiration d'un patient lorsque celui-ci bénéficie d'une thérapie respiratoire à haut débit, le procédé comprenant :
la détection d'une pression dans une lumière de détection d'une canule nasale à l'aide d'un capteur de pression positionné à une extrémité proximale de la lumière de détection opposée à une pointe de la lumière de détection, la lumière de détection étant positionnée de telle sorte que, pendant l'inspiration et l'expiration du patient, le gaz s'écoule sur la pointe de la lumière de détection pour créer un changement de pression à l'intérieur de la lumière de détection ;
l'émission du capteur de pression à un dispositif de commande d'un signal indicatif d'une pluralité de pressions détectées par le capteur de pression sur une période donnée ;
le traitement du signal à l'aide d'un algorithme stocké dans une mémoire du dispositif de commande, **caractérisé en ce que**,
le traitement du signal comprend l'identification des pics dans la pluralité de pressions détectées au fil du temps et la détermination d'une fréquence respiratoire actuelle ;
l'affichage de la fréquence respiratoire actuelle ;
la détermination d'une pression moyenne dans les données ; la détermination des pics maximum et minimum dans les données et/ou le filtrage des données ;
la détermination d'un pic maximum moyen sur une période donnée ;
la détermination d'un pic minimum moyen sur une période donnée ;
la détermination d'un seuil de pression en pourcentage du pic maximum moyen ; et
la détermination d'une respiration lorsque la pression :
passe d'au-dessous de la pression moyenne à au-dessus de la pression moyenne, puis
dépasse la pression moyenne d'au moins le seuil de pression, atteint un pic maximum, puis
passe d'au-dessus de la pression moyenne à au-dessous de la pression moyenne, puis
atteint un pic minimum, puis passe d'au-dessous de la pression moyenne à au-dessus de la pression moyenne.

15. Procédé selon la revendication 14, dans lequel la pression détectée est une pression différentielle entre une pression à l'intérieur de la lumière de détection et une pression ambiante.
